# EUROPÄISCHE PATENTSCHRIFT

(11) **EP 2 736 554 B1**
(45) Veröffentlichungstag und Bekanntmachung des Hinweises auf die Patenterteilung: **25.04.2018**
(21) Anmeldenummer: 12745627.5
(22) Anmeldetag: 27.07.2012
(51) Int. Cl.: A61M 1/16

(54) **VERFAHREN ZUM ENTFERNEN VON FLUID AUS EINEM BLUTFILTER NACH BEENDIGUNG EINER BLUTBEHANDLUNGSSITZUNG UND BEHANDLUNGSVORRICHTUNG ZUM DURCHFÜHREN DESSELBEN**
METHOD FOR REMOVING FLUID FROM A BLOOD FILTER AFTER A BLOOD TREATMENT SESSION HAS ENDED, AND TREATMENT DEVICE FOR CARRYING OUT THE METHOD
PROCÉDÉ PERMETTANT D'ÉLIMINER UN FLUIDE RESTANT DANS UN FILTRE SANGUIN À L'ISSUE D'UNE SÉANCE DE TRAITEMENT SANGUIN ET DISPOSITIF DE TRAITEMENT METTANT EN UVRE LEDIT PROCÉDÉ

(30) Priorität: 29.07.2011 DE 102011108785; 29.07.2011 US 201161512929 P
(43) Veröffentlichungstag der Anmeldung: 04.06.2014
(73) Patentinhaber: Fresenius Medical Care Deutschland GmbH, 61352 Bad Homburg (DE)
(72) Erfinder: BLASEK, Marco, 97464 Niederwerrn (DE); NOACK, Joachim, 97616 Bad Neustadt (DE)
(74) Vertreter: Bobbert & Partner Patentanwälte PartmbB
(86) Internationale Anmeldenummer: PCT/EP2012/003193
(87) Internationale Veröffentlichungsnummer: WO 2013/017239

(56) Entgegenhaltungen:
- WO-A1-96/40313
- DE-A1-102006 012 087
- US-A- 4 444 597

## Beschreibung

Die vorliegende Erfindung betrifft eine Steuerungs- und/oder Regelungseinrichtung zur Ausführung eines Verfahrens zum Entfernen von Fluid aus einem Blutfilter für die extrakorporale Blutbehandlung eines Patienten nach Beendigung der Blutbehandlungssitzung gemäß dem Oberbegriff des Anspruchs 1. Sie betrifft ferner eine medizinische Behandlungsvorrichtung gemäß Anspruch 14.

Blutfilter und extrakorporale Blutkreisläufe sind üblicherweise Einwegartikel und werden nach ihrer Verwendung entsorgt. Die Entsorgung ist kostenintensiv und nach Abfallgewicht zu vergüten. Aus diesem Grund, und auch zur Verringerung einer Kontaminationsgefahr, werden diese Einwegartikel daher vor ihrer Entsorgung von Fluid und insbesondere von Blut entleert. DE 10 2006 012 087 beschreibt ein Verfahren zum zumindest teilweisen Entleeren eines extrakorporalen Blutkeislaufs. Es ist Aufgabe der vorliegenden Erfindung, ein weiteres Verfahren sowie eine geeignete Vorrichtung zum Entfernen von Fluid aus einem Blutfilter oder einer Dialysatkammer hiervon und ggf. aus einem hiermit verbundenen extrakorporalen Blutkreislauf anzugeben.

Die erfindungsgemäße Aufgabe wird durch eine Regel- und/oder Steuerungseinrichtung mit den Merkmalen des Anspruchs 1 und der medizinischen Behandlungsvorrichtung mit den Merkmalen des Anspruchs 14 gelöst.

Es wird ein Verfahren zum Entfernen von Fluid aus einem zur Blutbehandlung eines Patienten verwendeten Blutfilter nach Beendigung der Blutbehandlungssitzung vorgeschlagen. Dabei weist der Blutfilter eine Blutkammer und eine Dialysatkammer auf, zwischen welchen eine Membran angeordnet ist, wobei die Blutkammer zur Blutbehandlung mit einer zur Blutkammer führenden arteriellen Blutleitung und einer von der Blutkammer weg führenden venösen Blutleitung sowie einer zur Dialysatkammer führenden Dialysatzulaufleitung und einer von der Dialysatkammer weg führenden Dialysatablaufleitung verbunden ist. Das erfindungsgemäße Verfahren umfasst das Verdrängen des Fluids aus der Dialysatkammer durch aktives oder passives Einbringen von Luft oder einer Flüssigkeit in die Dialysatzulaufleitung.

Die erfindungsgemäße Steuerungseinrichtung, welche auch als Regelungseinrichtung ausgestaltet sein kann, ist konfiguriert zur Durchführung des erfindungsgemäßen Verfahrens. Sie kann gegebenenfalls weitere Einrichtungen wie beispielsweise Speichereinrichtungen, Zugabeeinrichtungen, automatisierte Signalgebungseinrichtungen usw. umfassen.

Die erfindungsgemäße medizinische Behandlungsvorrichtung (im Folgenden auch kurz: Behandlungsvorrichtung) weist wenigstens einen extrakorporalen Blutkreislauf mit einem Leitungsinneren auf. Ferner ist sie versehen mit wenigstens einer an oder in dem extrakorporalen Blutkreislauf angeordneten Blutpumpe zum Fördern von Blut innerhalb des Leitungsinneren des extrakorporalen Blutkreislaufs. Zudem weist sie wenigstens eine Einrichtung, vorgesehen zum Einbringen von Luft in eine Dialysatzulaufleitung, welche während der Behandlung zum Zuführen von Dialysat zur Dialysatkammer des Blutfilters vorgesehen ist, auf.

Ein digitales Speichermedium, insbesondere in Form einer Diskette, CD oder DVD, USB-Stick, Flashcard, eine SD-Karte, EPROM und dergleichen, mit elektronisch auslesbaren Steuersignalen kann derart mit einem programmierbaren Computersystem zusammenwirken, dass die maschinellen Schritte des erfindungsgemäßen Verfahrens veranlasst werden.

Dabei können alle, einige oder manche der maschinell durchgeführten Schritte des erfindungsgemäßen Verfahrens veranlasst werden.

Ein Computerprogramm-Produkt weist einen volatilen, flüchtigen oder auf einem maschinenlesbaren Träger gespeicherten Programmcode zur Veranlassung der maschinellen Schritte des erfindungsgemäßen Verfahrens, wenn das Computerprogramm-Produkt auf einem Rechner abläuft, auf.

Der Begriff "maschinenlesbarer Träger", wie er hierin verwendet wird, bezeichnet in bestimmten Ausführungsformen der vorliegenden Erfindung einen Träger, der von Software und/oder Hardware interpretierbare Daten oder Informationen enthält. Der Träger kann ein Datenträger, wie eine Diskette, eine CD, DVD, ein USB-Stick, eine Flashcard, eine SD-Karte, ein EPROM und dergleichen sein.

Ein Computerprogramm weist einen Programmcode auf zur Veranlassung der maschinellen Schritte des erfindungsgemäßen Verfahrens, wenn das Computerprogramm auf einem Computer abläuft.

Ein insbesondere digitales, insbesondere nicht-flüchtiges, Speichermedium (hier auch als Träger bezeichnet), insbesondere in Form von Diskette, RAM, ROM, CD, Festplatte, DVD, USB-Stick, Flashcard, SD-Karte oder EPROM, insbesondere mit elektronisch oder optisch auslesbaren Steuersignalen, kann derart mit einem programmierbaren Computer oder Computersystem zusammenwirken, dass die maschinellen Schritte eines hierin beschriebenen erfindungsgemäßen Verfahrens veranlasst werden.

Dabei können alle, einige oder manche der maschinell durchgeführten Schritte des erfindungsgemäßen Verfahrens veranlasst werden.

Unter einem Computerprogramm-Produkt kann beispielsweise ein auf einem Träger gespeichertes Computerprogramm, ein Embedded System als umfassendes System mit einem Computerprogramm (z. B. ein elektronisches Gerät mit einem Computerprogramm), ein Netzwerk von computerimplementierten Computerprogrammen (z. B. Client/Serversystem, Cloud Computing System, etc.), oder ein Computer, auf dem ein Computerprogramm geladen ist, abläuft, gespeichert ist, ausgeführt oder entwickelt wird, verstanden werden.

Unter einem Computerprogramm kann beispielsweise ein physikalisches, vertriebsfähiges Software-Produkt verstanden werden, welches ein Programm aufweist.

Auch für das Computerprogramm-Produkt und das erfindungsgemäße Computerprogramm gilt, dass alle, einige oder manche der maschinell durchgeführten Schritte des erfindungsgemäßen Verfahrens veranlasst werden.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll eine erfindungsgemäße Ausführungsform erläutern.

Vorteilhafte Weiterentwicklungen der vorliegenden Erfindung sind jeweils Gegenstand von Unteransprüchen und Ausführungsformen.

Erfindungsgemäße Ausführungsformen können eines oder mehrere der im Folgenden genannten Merkmale aufweisen.

In manchen erfindungsgemäßen Ausführungsformen dient das Verfahren dem teilweisen, in anderen dem vollständigen Entfernen von Fluid aus einem zur Blutbehandlung eines Patienten verwendeten Blutfilter und/oder aus dem Blutkreislauf nach Beendigung der Blutbehandlungssitzung.

Der verwendete Blutfilter ist in einigen erfindungsgemäßen Ausführungsformen ein Hämodialysator.

Die zwischen Blutkammer und Dialysatkammer angeordnete Membran ist in bestimmten erfindungsgemäßen Ausführungsformen eine semipermeable Membran.

In gewissen erfindungsgemäßen Ausführungsformen führt die venöse Blutleitung von der Blutkammer zu einer venösen Konnektionsstelle.

In manchen Ausführungsformen wird statt Luft ein anderes Gas oder Gasgemisch verwendet, insbesondere in die Dialysatzulaufleitung eingebracht. In einigen Ausführungsformen erfolgt das Einbringen von Luft oder einer Flüssigkeit in die Dialysatzulaufleitung und/oder in die Dialysatkammer.

In bestimmten erfindungsgemäßen Ausführungsformen umfasst das Verfahren das Regeln oder Steuern des Einbringens von Luft unter Berücksichtigung von mittels wenigstens eines in oder an der Dialysatzulaufleitung oder in oder an der Dialysatablaufleitung vorgesehenen Drucksensors gemessenen Druckwerten.

Der wenigstens eine in oder an der Dialysatablaufleitung vorgesehene Drucksensor misst einen Dialysatdruck in einigen erfindungsgemäßen Ausführungsformen direkt, in anderen indirekt.

Das Verfahren umfasst in gewissen erfindungsgemäßen Ausführungsformen das Verschließen der arteriellen Blutleitung und/oder der venösen Blutleitung gegenüber der Atmosphäre oder das Gewährleisten eines solchen Verschlusses.

In bestimmten erfindungsgemäßen Ausführungsformen des Verfahrens wird wenigstens eine Patientenklemme zum Unterbinden einer Strömung von Blut innerhalb der arteriellen Blutleitung und/oder der venösen Blutleitung geschlossen. In einigen Ausführungsformen wird zusätzlich auch ein Single-Needle-Ventil geschlossen.

In manchen erfindungsgemäßen Ausführungsformen des Verfahrens wird eine arterielle Blutleitung nicht mit einer venösen Blutleitung verbunden zum Zwecke der Entleerung des Blutfilters. In gewissen erfindungsgemäßen Ausführungsformen unterbleibt eine Verbindung zwischen arterieller Blutleitung und venöser Blutleitung völlig.

In einigen erfindungsgemäßen Ausführungsformen wird die Verbindung zwischen arterieller Blutleitung und venöser Blutleitung, insbesondere ausschließlich zum Zwecke der Entleerung des Blutfilters erzeugt.

In manchen erfindungsgemäßen Ausführungsformen umfasst das Verfahren das Festlegen oder das Sicherstellen eines in der Blutkammer herrschenden Drucks oberhalb eines in der Dialysatkammer herrschenden Dialysatdrucks, insbesondere während jedes Zeitpunkts des Entleerungsvorganges.

In gewissen erfindungsgemäßen Ausführungsformen erfolgt das Verfahren ohne auch die Blutkammer von Fluid zu leeren, oder ohne die Blutkammer gleichzeitig zu leeren.

Hierdurch kann das Verfahren derart ablaufen, dass vorteilhafterweise kein Fluid über die Membran übertritt.

In erfindungsgemäßen Ausführungsformen des Verfahrens erfolgt das Entleeren der Dialysatkammer vor oder gleichzeitig mit dem Entleeren der Blutkammer oder des Blutkreislaufs. In manchen erfindungsgemäßen Ausführungsformen wird das Entleeren der Dialysatkammer abgeschlossen, bevor mit dem Entleeren der Blutkammer oder des Blutkreislaufs begonnen wird.

In einigen Ausführungsformen bedeutet das gleichzeitige Entleeren der Dialysatkammer mit dem Entleeren der Blutkammer oder des Blutkreislaufs ein zeitliches Überlappen. In manchen erfindungsgemäßen Ausführungsformen bedeutet Gleichzeitigkeit ein gleichzeitiges Beginnen und/oder ein gleichzeitiges Beenden.

In erfindungsgemäßen Ausführungsformen des Verfahrens wird Blut aus dem Blutfilter und/oder einem mit dem Blutfilter verbundenen extrakorporalen Blutkreislauf, welcher ein Leitungsinneres aufweist, entfernt. Dies erfolgt mittels wenigstens einer Blutpumpe der Behandlungsvorrichtung zum Fördern von Blut innerhalb des Leitungsinneren des extrakorporalen Blutkreislaufs sowie wenigstens einer zweiten Fördereinrichtung zum Einbringen wenigstens einer Substituatflüssigkeit in das Leitungsinnere des extrakorporalen Blutkreislaufs und/oder Fördern eines Leitungsinhalts innerhalb des Leitungsinneren des extrakorporalen Blutkreislaufs. Dies umfasst das Eintragen von Luft in das Leitungsinnere des extrakorporalen Blutkreislaufs durch Betreiben der Blutpumpe sowie das Einbringen von Substituatflüssigkeit in das Leitungsinnere des extrakorporalen Blutkreislaufs durch Betreiben der zweiten Fördereinrichtung.

In einigen erfindungsgemäßen Ausführungsformen des Verfahrens umfasst dieses das Erfassen einer qualitativen Änderung des Inhalts des Leitungsinneren des extrakorporalen Blutkreislaufs.

In bestimmten erfindungsgemäßen Ausführungsformen des Verfahrens umfasst dieses das Dekonnektieren des extrakorporalen Blutkreislaufs vom Gefäßsystem des Patienten.

In einigen erfindungsgemäßen Ausführungsformen des Verfahrens fördert die Blutpumpe ein definiertes Volumen an Luft.

In gewissen erfindungsgemäßen Ausführungsformen des Verfahrens wird ein "Luft-Blut-Inhalt" im Leitungsinneren des extrakorporalen Blutkreislaufs gefördert, bis eine "Luft-Blut-Grenze" eine Zugabestelle des extrakorporalen Blutkreislaufs für Substituatflüssigkeit in das Leitungsinnere erreicht. Ferner wird bei Erreichen eine Substituatflüssigkeit über die Zugabestelle in das Leitungsinnere des extrakorporalen Blutkreislaufs eingebracht.

In manchen erfindungsgemäßen Ausführungsformen des Verfahrens wird durch Betreiben der Fördereinrichtung eine vorbestimmte Substituatflüssigkeitsmenge in das Leitungsinnere des extrakorporalen Blutkreislaufs eingebracht.

In einigen erfindungsgemäßen Ausführungsformen des Verfahrens wird die Substituatflüssigkeit gefördert, bis die Erfassungseinrichtung Substituatflüssigkeit im Leitungsinneren des extrakorporalen Blutkreislaufs erfasst.

In bestimmten erfindungsgemäßen Ausführungsformen des Verfahrens wird der "Luft-Substituatflüssigkeit-Blut-Inhalt" durch Betreiben der Blutpumpe und/oder durch Betreiben der Fördereinrichtung entlang des Leitungsinneren des extrakorporalen Blutkreislaufs gefördert.

In gewissen erfindungsgemäßen Ausführungsformen des Verfahrens wird mittels der Fördereinrichtung zu wenigstens einem ersten Zeitpunkt Substituatflüssigkeit und zu wenigstens einem sich zeitlich vom ersten Zeitpunkt unterscheidenden zweiten Zeitpunkt Luft in das Leitungsinnere des extrakorporalen Blutkreislaufs eingebracht.

In manchen erfindungsgemäßen Ausführungsformen des Verfahrens ist die Erfassungseinrichtung mit vorgegebener Distanz zu der zweiten Zugangseinrichtung angeordnet. Das Verfahren umfasst in diesen Ausführungsformen ferner das Fördern des Inhalts des Leitungsinneren des extrakorporalen Blutkreislaufs über die vorgegebene Distanz bis zur Zugangseinrichtung, nachdem Luft an der Erfassungseinrichtung erkannt wurde.

In bestimmten erfindungsgemäßen Ausführungsformen des Verfahrens wird im Leitungsinneren des extrakorporalen Blutkreislaufs enthaltenes Blut über die zweite Zugangseinrichtung in das Gefäßsystem des Patienten eingebracht.

In einigen erfindungsgemäßen Ausführungsformen umfasst die Behandlungsvorrichtung wenigstens eine in einem Abschnitt des extrakorporalen Blutkreislaufs angeordnete Erfassungseinrichtung zum Erfassen wenigstens einer Änderung des Inhalts oder einer Eigenschaft des Inhalts des Leitungsinneren.

In manchen erfindungsgemäßen Ausführungsformen umfasst die Behandlungsvorrichtung wenigstens eine Fördereinrichtung zum Einbringen wenigstens einer Substituatflüssigkeit in das Leitungsinnere des extrakorporalen Blutkreislaufs und/oder zum Fördern eines Leitungsinhalts innerhalb des Leitungsinneren des extrakorporalen Blutkreislaufs.

Das Verfahren umfasst in einigen erfindungsgemäßen Ausführungsformen ferner das Eintragen oder Einbringen von Luft und Substituatflüssigkeit in den extrakorporalen Blutkreislauf, beispielsweise nach Beenden einer Blutbehandlungssitzung.

Eine solche "Blutbehandlungssitzung" kann beispielsweise eine Behandlungseinheit mittels Hämodialyse, Hämofiltration, Hämodiafiltration und/oder eines Zellseparationsverfahrens und auf die Behandlung und/oder Reinigung von Blut gerichtet sein. Zum Durchführen einer solchen Blutbehandlung wird eine geeignete Blutbehandlungsvorrichtung verwendet.

Eine zum Durchführen des erfindungsgemäßen Verfahrens geeignete Blutbehandlungsvorrichtung weist in manchen erfindungsgemäßen Ausführungsformen einen extrakorporalen Blutkreislauf mit einem Leitungsinneren, verschiedene Fördereinrichtungen zum Einbringen und/oder Fördern verschiedener Fluide im Leitungsinneren des extrakorporalen Blutkreislaufs, und beispielsweise eine Einrichtung zum Behandeln des Bluts des Patienten, wie einen oder mehrere Blutfilter und/oder einen oder mehrere Dialysatoren und/oder einen oder mehrere Adsorber, auf oder ist hiermit verbunden. Sie kann ferner Behälter zum Aufbewahren von Fluiden, Elemente zum Einbringen der Fluide, wie beispielsweise Schlauchelemente und/oder Ventile, sowie weitere Einrichtungen, wie z. B. eine Luftabscheidekammer zum Entfernen von Luft aus dem Blut während der Blutbehandlung und/oder Sensoren und/oder Detektoren zum Erfassen verschiedener relevanter Parameter, wie beispielsweise einen Druck im extrakorporalen Blutkreislauf, aufweisen.

Fördereinrichtungen schließen Membranpumpen, Schlauchpumpen, Rollenpumpen usw. ein. Die Blutpumpe kann z. B. als Schlauchpumpe oder Rollenpumpe ausgeführt sein.

Als Fördereinrichtung zum Einbringen wenigstens einer Substituatflüssigkeit in das Leitungsinnere des extrakorporalen Blutkreislaufs und/oder Fördern eines Leitungsinhalts innerhalb des Leitungsinneren des extrakorporalen Blutkreislaufs kann eine peristaltische Pumpe, z. B. eine Rollenpumpe, eingesetzt werden. Es kann aber auch ein anderer Pumpentyp, z. B. eine Membranpumpe, insbesondere eine hochgenau dosierende Membranpumpe, eingesetzt werden.

Bei dieser Fördereinrichtung kann es sich um eine "zweite" Fördereinrichtung handeln, also eine Fördereinrichtung, welche nicht mit der Blutpumpe identisch ist. Die Blutpumpe kann jedoch auch derart ausgestaltet sein, dass sie sowohl die für eine Blutpumpe typische Funktion ausführt als auch die Funktion des Einbringens von Substituatflüssigkeit in das Leitungsinnere und/oder das Fördern von Leitungsinhalt erfüllt. Wenn im Folgenden allein der besseren Lesbarkeit wegen von einer "zweiten" Fördereinrichtung die Rede ist, so ist darunter die Blutpumpe oder eine sich hiervon unterscheidende Fördereinrichtung zu verstehen. Beide Varianten sind gleichermaßen von der vorliegenden Erfindung umfasst.

Das erfindungsgemäße Verfahren umfasst den Schritt des Einbringens oder Eintragens von Luft in das Leitungsinnere des extrakorporalen Blutkreislaufs durch Betreiben der Blutpumpe. Die Luft kann beispielsweise Atmosphärenluft sein.

Das "Eintragen von Luft in das Leitungsinnere des extrakorporalen Blutkreislaufs" nach Beenden der Blutbehandlungssitzung kann ausschließlich oder unterstützend durch die Blutpumpe, durch die Fördereinrichtung oder durch die Druckluftquelle erfolgen.

Kombinationen der vorgenannten Optionen sind ebenfalls umfasst, ebenso ein passives Eindringenlassen von Luft.

Das "Einbringen von Substituatflüssigkeit in das Leitungsinnere des extrakorporalen Blutkreislaufs" erfolgt durch Betreiben der Blutpumpe und/oder der zweiten Fördereinrichtung.

Die Blutpumpe kann Substituatflüssigkeit fördern, indem sie selbige aus einer Zuleitung zu einem Behälter für die Substituatflüssigkeit ansaugt, die zwischen der Saugseite der Blutpumpe und einer Erfassungseinrichtung in den extrakorporalen Blutkreislauf mündet. Hierzu kann beispielsweise eine im arteriellen Zweig des extrakorporalen Blutkreislaufs vorgesehene Schlauchklemme vorgesehen sein.

Ist vorgesehen, dass die Blutpumpe sowohl Blut als auch Substituatflüssigkeit in den extrakorporalen Blutkreislauf einbringt und fördert, kann das erfindungsgemäße Verfahren mit nur einer Pumpe durchgeführt werden. Obwohl eine solche weiter bevorzugte Ausführungsform von der vorliegenden Erfindung umfasst ist, werden im Folgenden Ausführungsformen beschrieben, bei denen eine Blutpumpe und eine zweite Fördereinrichtung eingesetzt werden. Die folgende Beschreibung soll ein Verständnis der der vorliegenden Erfindung zugrunde liegenden Prinzipien und Funktionen der einzelnen Komponenten vereinfachen.

Eine "Substituatflüssigkeit" kann dabei beispielsweise jede zur Verwendung bei einer Blutbehandlung, wie z. B. einer Hämodiafiltration, allgemein bekannte Substituatflüssigkeit sein, vorzugsweise eine bereits während der Blutbehandlungssitzung verwendete und somit über eine Fluidverbindung bereits in den extrakorporalen Blutkreislauf eingebundene oder einbringbare Lösung oder isotone Kochsalzlösung, wie z. B. eine 0,9%-ige NaCl-Lösung.

Beim erfindungsgemäßen Verfahren werden sowohl Luft als auch Substituatflüssigkeit bzw. ein beliebiges, im Rahmen der vorliegenden Erfindung sinnvoll verwendbares Fluid in das Leitungsinnere des extrakorporalen Blutkreislaufs nach Beenden einer Blutbehandlungssitzung eingebracht oder eingetragen. Die Substituatflüssigkeit kann dabei unter anderem bevorzugt eine Spülfunktion des Leitungsinneren zum Vorbeugen eines Kontaminationsrisikos ausüben.

Sie kann weiter bevorzugt eine Schaumbildung z. B. am Ausgang des Blutfilters verringern oder verhindern.

Die Luft kann unter anderem bevorzugt Flüssigkeiten wie Blut aus dem Leitungsinneren des extrakorporalen Blutkreislaufs und/oder dem Blutfilter verdrängen und somit das Gewicht des zu entsorgenden Blutfilters oder des extrakorporalen Blutkreislaufs verringern.

In einer bevorzugten erfindungsgemäßen Ausführungsform ist das Erfassen einer qualitativen Änderung des Inhalts des Leitungsinneren des extrakorporalen Blutkreislaufs mit Hilfe wenigstens einer in einem Abschnitt des extrakorporalen Blutkreislaufs angeordneten Erfassungseinrichtung umfasst.

Die "qualitative Änderung" kann sich auf einen oder mehrere Bereiche oder Abschnitte des extrakorporalen Blutkreislaufs beziehen, beispielsweise einen Bereich oder Abschnitt, in welchem die Erfassungseinrichtung vorliegt.

Eine "qualitative Änderung des Inhalts des Leitungsinneren" schließt eine Änderung der Zusammensetzung des Inhalts des Leitungsinneren, wie beispielsweise eine Änderung der einzelnen Anteile an Blut, Substituatflüssigkeit und/oder Luft im Leitungsinneren oder einem Abschnitt hiervon, bezogen aufeinander, ein. Auch das Fehlen eines vormals vorhandenen Fluids kann eine Änderung der Zusammensetzung darstellen. Eine qualitative Änderung kann auch ein Übergang, z. B. von einem gasförmigen Inhalt zu einem flüssigen Inhalt, oder umgekehrt sein. Dies kann z. B. ein Übergang von Blut zu Luft sein. Ebenso kann eine qualitative Änderung ein Übergang von einem ersten flüssigen Inhalt zu einem zweiten, sich unterscheidenden flüssigen Inhalt sein, wie beispielsweise ein Übergang von Blut zu Substituatflüssigkeit. Solche Änderungen können z. B. leicht anhand einer optischen Änderung des Inhalts, wie einer Aufhellung oder einer Verdunkelung des Inhalts, erfasst werden.

Die in einem Abschnitt des extrakorporalen Blutkreislaufs angeordnete "Erfassungseinrichtung" kann z. B. ein optischer Sensor sein, der eine optische Änderung des Inhalts des Leitungsinneren oder eine Eigenschaft des Inhalts erfasst. So ist z. B. ein Blut-Luft-Inhalt im Leitungsinneren des extrakorporalen Blutkreislaufs aufgrund des vorhandenen Sauerstoffs heller als reines Blut. Weitere geeignete Sensoren schließen Drucksensoren zum Erfassen eines Druckabfalls bei einer Änderung des Inhalts im Leitungsinneren und Sensoren zum Erfassen einer Änderung der Dichte des Inhalts des Leitungsinneren des extrakorporalen Blutkreislaufs ein, ohne darauf beschränkt zu sein.

Der "Abschnitt des extrakorporalen Blutkreislaufs" kann ein arterieller und/oder venöser Abschnitt des extrakorporalen Blutkreislaufs sein. Der "arterielle Abschnitt" bezeichnet einen Abschnitt des extrakorporalen Blutkreislaufs, durch den das Blut aus dem Gefäßsystem des Patienten in Richtung zur Blutbehandlungseinrichtung strömt. Der "venöse Abschnitt" bezeichnet den Abschnitt des extrakorporalen Blutkreislaufs, durch den das Blut aus der Blutbehandlungseinrichtung zurück zum Gefäßsystem des Patienten strömt.

Die Erfassungseinrichtung kann Auskunft geben über die momentanen Verhältnisse im Leitungsinneren des extrakorporalen Blutkreislaufs an einem Abschnitt hiervon. Das erfindungsgemäße Verfahren kann somit besser und gezielter steuerbar bzw. regelbar sein. Ferner können auch mehrere Erfassungseinrichtungen zum Erfassen gleicher Parameter in verschiedenen Abschnitten des extrakorporalen Blutkreislaufs und/oder verschiedene Erfassungseinrichtungen zum Erfassen verschiedener Parameter in gleichen Abschnitten des extrakorporalen Blutkreislaufs in selbigem angeordnet sein.

In einer ebenfalls bevorzugten Weiterentwicklung des erfindungsgemäßen Verfahrens umfasst der extrakorporale Blutkreislauf wenigstens eine mit einem Abschnitt des Gefäßsystems des Patienten konnektierbare Zugangseinrichtung, und das Verfahren umfasst ein Dekonnektieren des extrakorporalen Blutkreislaufs vom Gefäßsystem des Patienten, insbesondere im Bereich einer ersten, beispielsweise arteriellen, Zugangseinrichtung, insbesondere an einem Ende des extrakorporalen Blutkreislaufs.

Das "Dekonnektieren des extrakorporalen Blutkreislaufs vom Gefäßsystem des Patienten" bedeutet das Unterbrechen einer Verbindung zwischen dem extrakorporalen Blutkreislauf und dem Gefäßsystem des Patienten in einem Abschnitt des extrakorporalen Blutkreislaufs, beispielsweise an einem Ende hiervon. Dabei kann die Unterbrechung sowohl am arteriellen als auch am venösen Abschnitt erfolgen, wobei in der vorliegenden Erfindung ein Dekonnektieren des arteriellen Abschnitts des extrakorporalen Blutkreislaufs bevorzugt ist.

Unter einem Dekonnektieren im "Bereich der ersten Zugangseinrichtung" kann z. B. das Herausziehen der arteriellen Konnektionsnadel eines Double-Needle-Zugangs verstanden werden.

Unter einem Dekonnektieren kann auch das Unterbrechen der Strömungsverbindung zwischen dem arteriellen Abschnitt des extrakorporalen Blutkreislaufs und der arteriellen Konnektionsnadel verstanden werden.

Bei der Single-Needle-Variante kann hierunter das Unterbrechen der Verbindung zwischen dem arteriellen Schenkel des "Y"-förmigen Abschnitts des extrakorporalen Blutkreislaufs und der einzigen mit dem Gefäßsystem des Patienten verbundenen Konnektionsnadel verstanden werden. Das offene Lumen des arteriellen Schenkels des Y-Stücks kann nach seinem Abtrennen auf beliebige Weise (manuell, maschinell, automatisch, usw.) verschlossen werden.

Alternativ oder zusätzlich kann gleiches auch für den venösen Abschnitt des extrakorporalen Blutkreislaufs und den venösen Zugang zum Gefäßsystem des Patienten angewandt werden.

Die Auswahl der Zugangseinrichtung mit geeignetem Zugang zum Gefäßsystem des Patienten ist für das Ausführen der vorliegenden Erfindung nicht entscheidend. Der Einfachheit halber wird hier des Öfteren auf den Double-Needle-Zugang verwiesen, ohne die vorliegende Erfindung darauf einschränken zu wollen. Es ist zu beachten, dass die Erfindung in gleicher Weise mit einem Single-Needle-Zugang oder jeder weiteren, zum Zwecke einer Blutbehandlung geeigneten Zugangseinrichtung ausführbar ist.

Das Dekonnektieren des extrakorporalen Blutkreislaufs ermöglicht auf einfache und unkomplizierte Weise das Eintragen oder Einbringen von Luft in das Leitungsinnere des extrakorporalen Blutkreislaufs zum Ausführen des erfindungsgemäßen Verfahrens durch eine beim Dekonnektieren hergestellte Fluidkommunikation mit der Atmosphäre.

Eine weiter bevorzugte Ausführungsform der vorliegenden Erfindung schließt das Fördern eines definierten Volumens an Luft durch die Blutpumpe ein.

Um ein "definiertes Volumen an Luft" zu fördern, kann die Blutpumpe beispielsweise für eine vorher festgelegte Zeitdauer und/oder eine bestimmte Anzahl an Umdrehungen betrieben werden.

Das "Fördern eines definierten Volumens an Luft" kann aber auch dem Fördern eines bestimmen Fördervolumens und/oder dem Fördern über eine vorbestimmte Förderstrecke des Inhalts entlang des Leitungsinneren des extrakorporalen Blutkreislaufs entsprechen.

Eine weiter bevorzugte Ausführungsform des erfindungsgemäßen Verfahrens umfasst das Fördern des "Luft-Blut-Inhalts" im Leitungsinneren des extrakorporalen Blutkreislaufs, bis der "Luft-Blut-Inhalt" eine Zugabestelle des extrakorporalen Blutkreislaufs für Substituatflüssigkeit in das Leitungsinnere des extrakorporalen Blutkreislaufs erreicht.

Der "Luft-Blut-Inhalt" kann z. B. in Richtung weg vom dekonnektierten Ende des extrakorporalen Blutkreislaufs gefördert werden.

Der "Luft-Blut-Inhalt" bezeichnet den Inhalt, der durch Einbringen von Luft in den nach Beenden der Blutbehandlungssitzung bereits Blut enthaltenden extrakorporalen Blutkreislauf erzeugt wird, wobei Blut und Luft die einzigen oder im Wesentlichen die einzigen Fluide sein können, die im Inneren des extrakorporalen Blutkreislauf vorliegen.

Eine "Zugabestelle des extrakorporalen Blutkreislaufs für Substituatflüssigkeit in das Leitungsinnere des extrakorporalen Blutkreislaufs" kann in dem arteriellen und/oder dem venösen Abschnitt des extrakorporalen Blutkreislaufs angeordnet sein. Bevorzugt ist die "Zugabestelle" in einem Abschnitt des extrakorporalen Blutkreislaufs angeordnet, den das Blut in Richtung weg vom dekonnektierten Ende des extrakorporalen Blutkreislaufs durchströmt. Besonders bevorzugt ist die Zugabestelle in dem Abschnitt des extrakorporalen Blutkreislaufs angeordnet, den das Blut vor Erreichen und Durchströmen der Blutbehandlungseinrichtung, wie beispielsweise eines Blutfilters, durchströmt. Eine solche Zugabestelle kann in geeigneter Weise zum Zugeben von Substituatflüssigkeit in den extrakorporalen Blutkreislauf ausgewählt sein, derart, dass Substituatflüssigkeit als eine Art Trennschicht oder als eine Art Trennvolumen zwischen das Blutvolumen und das Luftvolumen mittels der zweiten Fördereinrichtung eingebracht werden kann.

Geeignete Beispiele für eine Zugabestelle schließen ein Öffnungs-/Verschlussventil, einen Absperrhahn, eine zuschaltbare Nebenleitung eines verzweigten Abschnitts des extrakorporalen Blutkreislaufs usw. ein.

Das "Fördern des Luft-Blut-Inhalts" kann durch Betreiben der Blutpumpe erfolgen. Wenn der Luft-Blut-Inhalt bzw. eine sich ausbildende Luft-Blut-Grenze oder ein Luft-Blut-Übergangs- bzw. Mischbereich die Zugabestelle des extrakorporalen Blutkreislaufs für Substituatflüssigkeit in das Leitungsinnere des extrakorporalen Blutkreislaufs erreicht, kann das Fördern des Luft-Blut-Inhalts gestoppt werden. Dies kann durch Stoppen der Blutpumpe erreicht werden. Das exakte Stoppen des Förderns des Luft-Blut-Inhalts an oder um eine vorgegebene Distanz vor der Zugabestelle für Substituatflüssigkeit kann durch das Einbringen eines definierten Volumens an Luft erreicht werden.

Unter dem "Erreichen" der Zugabestelle ist erfindungsgemäß vorzugsweise und beispielhaft zu verstehen, dass der Luft-Blut-Inhalt bis zu einer Position in Nähe oder in unmittelbarer Nähe der Zugabestelle (Mündung, Einstrombereich oder dgl.) für die Substituatflüssigkeit - z. B. davor - oder bis exakt an die Position der Zugabestelle für Substituatflüssigkeit gefördert wird. Der Begriff "Erreichen" soll daher nicht auf ein absolutes "Ankommen" des Luft-Blut-Inhalts an der Zugabestelle für Substituatflüssigkeit beschränkt sein. Vielmehr soll er eine relative oder anderweitig geeignete Position des geförderten Leitungsinneren bezüglich der Zugabestelle für Substituatflüssigkeit definieren. Diese Definition soll in ihrer Breite ferner für alle hier angegebenen Ausführungsformen gelten.

So kann in einer bevorzugten Ausführungsform die Blutpumpe angehalten werden, wenn die Luft-Blut-Grenze die Zugabestelle noch nicht erreicht hat, also davor. Dies kann vorteilhaft und vorzugsweise derart erfolgen, dass keine Luft in einen Blutfilter gefördert wird.

Von der Erfindung umfasst ist auch eine Anordnung, bei welcher das Fördern von Substituatflüssigkeit beginnt, noch während die Blutpumpe Luft ansaugt, die Luft die Zugabestelle für die Substituatflüssigkeit aber noch nicht erreicht hat. Auf diese Weise kann vorteilhaft ein "Freispülen" des Blutfilters erfolgen.

Daneben kann zusätzlich eine weitere Erfassungseinrichtung, wie beispielsweise ein geeignet ausgebildeter Luft-Blutdetektor, in dem Abschnitt des extrakorporalen Kreislaufs, wie beispielsweise dem arteriellen Abschnitt des extrakorporalen Blutkreislaufs, z. B. zwischen der Blutpumpe und der Zugabestelle für Substituatflüssigkeit, angeordnet sein und das Auftreten von Luft im Leitungsinneren des extrakorporalen Blutkreislaufs erfassen. Ein solcher Luft-Blutdetektor kann wiederum als optischer Sensor ausgebildet sein und das Auftreten von Luft im Leitungsinneren des extrakorporalen Blutkreislaufs als optische Änderung des Leitungsinhalts erfassen.

Wenn der Luft-Blut-Inhalt die Zugabestelle für Substituatflüssigkeit erreicht und/oder eine Erfassungseinrichtung das Auftreten von Luft im Leitungsinneren des extrakorporalen Blutkreislaufs erfasst, wird durch Betreiben der zweiten Fördereinrichtung, zum Beispiel einer Schlauch- oder Rollenpumpe, Substituatflüssigkeit an der Zugabestelle für Substituatflüssigkeit in das Leitungsinnere des extrakorporalen Blutkreislaufs eingetragen oder eingebracht.

Die Förderung von Substituatflüssigkeit kann vorzugsweise bereits beginnen, bevor die Blutpumpe Luft ansaugt. Alternativ dazu kann sie jedoch vorzugsweise auch beginnen, während die Blutpumpe Luft ansaugt.

Bei der in das Leitungsinnere des extrakorporalen Blutkreislaufs eingetragenen oder eingebrachten Substituatflüssigkeit kann es sich um eine unbestimmte Menge oder um eine vorbestimmte Menge oder um eine begrenzte Menge an Substituatflüssigkeit handeln.

Das "Einbringen einer vorbestimmten Substituatflüssigkeitsmenge" kann einen "Luft-Substituatflüssigkeit-Blut-Inhalt" erzeugen. Der Begriff "Luft-Substituatflüssigkeit-Blut-Inhalt" bezeichnet hierbei, dass die Substituatflüssigkeit zwischen das durch den extrakorporalen Blutkreislauf geförderte Blut und die durch den extrakorporalen Blutkreislauf geförderte Luft eingebracht wird und so eine Art "Puffer" zwischen Luft und Blut bildet.

Eine "vorbestimmte Substituatflüssigkeitsmenge" kann einem bestimmten Fördervolumen und/oder einer bestimmten Förderstrecke des Inhalts entlang des Leitungsinneren des extrakorporalen Blutkreislaufs entsprechen und beispielsweise durch Betreiben einer hochgenau dosierenden Membranpumpe erfolgen.

Die Substituatflüssigkeitsmenge kann vorzugsweise als Größe, zum Beispiel als Volumen mit vorgegebener Maßzahl und Einheit, vorbestimmt sein. Die absolute Größe der Substituatflüssigkeitsmenge kann vorzugsweise beispielsweise in einer Steuereinrichtung der erfindungsgemäßen Behandlungsvorrichtung hinterlegt und/oder eingebbar sein. Die Substituatflüssigkeitsmenge kann vorzugsweise im Rahmen der technischen Genauigkeit exakt gefördert werden.

Um eine exakte Menge an Substituatflüssigkeit vorzubestimmen, können zum Beispiel technische Spezifikationen des eingesetzten extrakorporalen Blutkreislaufs, wie beispielsweise die Innenvolumina des Schlauchsatzes, in der Steuereinrichtung gespeichert oder in diese eingegeben werden. Anhand der technischen Spezifikationen der einzelnen Komponenten des extrakorporalen Blutkreislaufs können zum Beispiel eine erforderliche Förderzeit und/oder ein Fördervolumen berechnet werden.

Eine "begrenzte Substituatflüssigkeitsmenge" kann eine, zum Beispiel anhand von Erfahrungswerten des Bedienpersonals ausgewählte, Menge an Substituatflüssigkeit sein. Vorzugsweise kann eine begrenzte Menge an Substituatflüssigkeit eingebracht und so lange gefördert werden, bis an einer weiteren Erfassungseinrichtung das Auftreten von Substituatflüssigkeit im Leitungsinneren des extrakorporalen Blutkreislaufs erfasst wird. Eine begrenzte Substituatflüssigkeitsmenge muss daher nicht genau bekannt sein und/oder einem bestimmten Fördervolumen entsprechen. Eine begrenzte Substituatflüssigkeitsmenge kann jedoch indirekt durch das Innenvolumen der von der Substituatflüssigkeitsmenge durchströmten Komponenten des extrakorporalen Blutkreislaufs, insbesondere das Innenvolumen des Abschnitts von der Zugabestelle für Substituatflüssigkeit und/oder der Blutbehandlungseinrichtung bis zu einer weiteren Erfassungseinrichtung begrenzt sein. Das Volumen ist dabei somit bestimmt im Sinne von "begrenzt", ohne jedoch exakt bekannt zu sein, und ohne beispielsweise in Millilitern angegeben werden zu können und/oder ohne in einer Steuerung hinterlegt worden oder eingebbar zu sein. Das Einbringen einer begrenzten Substituatflüssigkeitsmenge kann zum Beispiel von Vorteil sein, wenn der Filtertyp einer Blutbehandlungsvorrichtung oder dessen Fassungsvermögen nicht bekannt oder nicht richtig angegeben ist.

Dabei kann die Substituatflüssigkeit aus einem dafür vorgesehenen Vorratsbehälter über entsprechende Leitungssysteme des extrakorporalen Blutkreislaufs an der Zugabestelle für die Substituatflüssigkeit in den extrakorporalen Blutkreislauf eingebracht werden.

Der auf diese Weise erzeugte "Luft-Substituatflüssigkeit-Blut-Inhalt" kann in Richtung weg vom dekonnektierten Ende durch Betreiben der Blutpumpe und/oder durch Betreiben der zweiten Fördereinrichtung gefördert werden.

Nach Einbringen von Substituatflüssigkeit und somit Erzeugen des "Luft-Substituatflüssigkeit-Blut-Inhalts" kann ferner weiter Luft in das Leitungsinnere des extrakorporalen Blutkreislaufs eingebracht werden. Dies kann insbesondere durch Betreiben der Blutpumpe und/oder durch Betreiben der zweiten Fördereinrichtung erfolgen. Das Ansaugen und Einbringen von Luft durch die zweite Fördereinrichtung kann zum Beispiel durch Herstellen einer Verbindung zwischen der Saugseite der zweiten Fördereinrichtung und einem Äußeren des extrakorporalen Blutkreislaufs, z. B. der Atmosphäre, erfolgen.

Das weitere Einbringen von Luft kann insbesondere dazu dienen, das Leitungsinnere des extrakorporalen Blutkreislaufs im Wesentlichen von im Inneren vorhandenen Fluiden, insbesondere Blut und Substituatflüssigkeit, zu befreien oder die hiervon im Leitungsinneren vorliegenden Mengen zu verringern.

Das Fördern des "Luft-Substituatflüssigkeit-Blut-Inhalts" kann beispielsweise dann gestoppt werden, wenn die Erfassungseinrichtung Substituatflüssigkeit im Leitungsinneren des extrakorporalen Blutkreislaufs erkennt.

Die "Erfassungseinrichtung" ist wie vorstehend definiert und kann beispielsweise im venösen Abschnitt des extrakorporalen Blutkreislaufs, z. B. zwischen der Blutbehandlungseinrichtung und der venösen Zugangseinrichtung zum Gefäßsystem des Patienten und insbesondere zwischen einer Tropfkammer im venösen Abschnitt und der venösen Zugangseinrichtung, angeordnet sein.

Die Erfassungseinrichtung kann das Auftreten von Substituatflüssigkeit in einem bestimmten Abschnitt des Leitungsinneren des extrakorporalen Blutkreislaufs beispielsweise anhand einer optischen Änderung des Inhalts des Leitungsinneren erkennen.

Wenn die Erfassungseinrichtung das Auftreten von Luft oder Substituatflüssigkeit in dem Leitungsinneren des extrakorporalen Blutkreislaufs erkennt, kann das Fördern des "Luft-Substituatflüssigkeit-Blut-Inhalts" gestoppt werden.

Dies kann durch Stoppen der zweiten Fördereinrichtung erfolgen.

Ferner ist in einer weiteren Ausführungsform des erfindungsgemäßen Verfahrens bevorzugt, die Erfassungseinrichtung mit vorgegebener Distanz zu einer zweiten Zugangseinrichtung anzuordnen und den Inhalt des Leitungsinneren über die vorgegebene Distanz bis zur Zugangseinrichtung zu fördern, nachdem Luft an der Erfassungseinrichtung erkannt wurde.

Die "zweite Zugangseinrichtung" kann, ohne darauf beschränkt zu sein, eine venöse Konnektionsnadel eines Double-Needle-Zugangs oder ein "venöser" Schenkel eines Y-Stückes sein.

Wenn von der Erfassungseinrichtung Luft erkannt wurde, kann der "Luft-Substituatflüssigkeit-Blut-Inhalt" über die vorgegebene Distanz bis zur zweiten Zugangseinrichtung gefördert werden und so Blut und gegebenenfalls auch Substituatflüssigkeit aus dem extrakorporalen Blutkreislauf entfernt werden. Dabei kann das Blut lediglich aus dem extrakorporalen Blutkreislauf entleert oder zugleich in das Gefäßsystem des Patienten rückgeführt werden.

In manchen erfindungsgemäßen Ausführungsformen umfasst das Verfahren ein - vorzugsweise vollständiges oder anteiliges - Entleeren der Dialysatkammer und/oder der Diaylsierflüssigkeits- oder Dialysatleitungen über die Membran des Blutfilters hinweg in die Blutkammer des Blutfilters hinein nicht.

In einer weiter bevorzugten Ausführungsform des erfindungsgemäßen Verfahrens wird das im Leitungsinneren des extrakorporalen Blutkreislaufs enthaltene Blut - insbesondere im Wesentlichen vollständig - über die zweite Zugangseinrichtung in das Gefäßsystem des Patienten rückgeführt. Der Begriff "im Wesentlichen vollständig rückgeführt" meint hierbei, dass das im Leitungsinneren des extrakorporalen Blutkreislaufs vorhandene Blut nahezu rückstandsfrei aus dem extrakorporalen Blutkreislauf entfernt wird. Die gegebenenfalls im extrakorporalen Blutkreislauf aus technischen Gründen wie Benetzungsverhalten oder in der Tropfkammer verbleibenden Blutrückstände sind dabei als vernachlässigbar gering anzusehen.

Das "Rückführen von Blut in das Gefäßsystem des Patienten" kann erfolgen, wenn ein Ende des extrakorporalen Blutkreislaufs, wie beispielsweise das Ende des venösen Abschnitts, z. B. die venöse Konnektionsnadel, mit dem Gefäßsystem des Patienten konnektiert ist. Diese Verbindung kann nach Beenden der Blutbehandlungssitzung beibehalten oder erneut hergestellt werden.

Daneben kann es weiter bevorzugt sein, auch die Substituatflüssigkeit, insbesondere im Wesentlichen vollständig, aus dem Leitungsinneren des extrakorporalen Blutkreislaufs zu entfernen. Dies kann durch Fördern des Inhalts des Leitungsinneren des extrakorporalen Blutkreislaufs lediglich über die vorgegebene Distanz zwischen der Erfassungseinrichtung und der Zugangseinrichtung erreicht werden, nachdem die Erfassungseinrichtung das Auftreten von Luft erkannt hat. Die vorgegebene Distanz kann mit dem durch die Blutpumpe eingetragenen definierten Fördervolumen an Luft korrelieren.

Das "im Wesentlichen vollständige Entfernen" der Substituatflüssigkeit aus dem Leitungsinneren des extrakorporalen Blutkreislaufs kann, wie vorstehend in Bezug auf das im Leitungsinneren enthaltene Blut beschrieben, als ebenfalls nahezu rückstandsfrei angesehen werden.

Ein vollständiges oder nahezu vollständiges Entfernen ist erfindungsgemäß jedoch nicht erforderlich, um Vorteile zu erzielen.

Nach dem kompletten Dekonnektieren des Patienten (arteriell und venös) könnte nach der Blutrückgabe an den Patienten optional noch eines der bekannten (halb-)automatisierten Verfahren zum mehr oder weniger kompletten Entleeren des Blutschlauchsatzes durchgeführt werden. Solche Verfahren fördern Flüssigkeitsreste z. B. in den sog. Rinse-Port der Dialysemaschine. Natürlich kann die restliche Flüssigkeit in jeden beliebigen Ablauf gefördert werden. Derartige Entleerungsverfahren können sich an das erfindungsgemäße Verfahren anschließen bzw. Teil einer Ausführungsform desselben sein. Sie sind jedoch zur Ausführung der Erfindung nicht erforderlich.

Da mit der erfindungsgemäßen Behandlungsvorrichtung das vorstehend beschriebene erfindungsgemäße Verfahren durchführbar ist, wird zur Vermeidung von Wiederholungen auf die vorstehend beschriebenen Ausführungen derselben verwiesen.

Eine Weiterentwicklung der erfindungsgemäßen Behandlungsvorrichtung sieht das Anordnen wenigstens einer Erfassungseinrichtung zum Erfassen wenigstens einer Änderung des Inhalts des Leitungsinneren des extrakorporalen Blutkreislaufs oder einer Eigenschaft des Inhalts in einem Abschnitt des extrakorporalen Blutkreislaufs vor. Bei einer Eigenschaft des Inhalts kann es sich um eine Zusammensetzung, eine physikalische, chemische oder biologische Größe, beispielsweise eine Transparenz, einen pH-Wert und vieles dergleichen mehr handeln. Eine derartige Erfassungseinrichtung kann der vorstehend beschriebenen entsprechen, so dass zur Vermeidung von Wiederholungen auf deren obige Beschreibung verwiesen wird. Umgekehrt kann das zur zweiten Erfassungseinrichtung Ausgeführte ungeschmälert auch auf jede andere hier mitoffenbarte Erfassungseinrichtung zutreffen.

Eine Behandlungsvorrichtung gemäß der vorliegenden Erfindung kann, ohne darauf beschränkt zu sein, zum Durchführen einer Hämodialyse, einer Hämofiltration, einer Hämodiafiltration und Separationsverfahren geeignet sein.

Ein erfindungsgemäß zu erzielender Vorteil besteht darin, dass einem Kontaminationsrisiko bei der weiteren Handhabung oder der Entsorgung des Blutfilters und/oder des extrakorporalen Blutkreislaufs vorgebeugt werden kann.

Ferner kann das Bruttoabfallgewicht des Blutfilters und/oder des extrakorporalen Blutkreislaufs verringert werden. Da eine Entsorgung von kontaminierten Einwegartikeln wie Blutfilter und extrakorporalem Blutkreislauf nach Gewicht berechnet wird, können somit in vorteilhafter Weise Kosten eingespart werden.

In bestimmten erfindungsgemäßen Ausführungsformen kann ein Entleeren der Dialysatkammer ablaufen, ohne dass dabei Fluid, insbesondere Luft, über die Membran übertritt und mit Inhalt des mit dem Patienten verbundenen Blutkreislaufs in Kontakt gelangt.

Die Entleerung wenigstens des Blutfilters kann in bestimmten Ausführungsformen der Erfindung ohne wesentliche oder ohne jede Interaktion des Benutzers, z. B. des Arztes, erfolgen, jedenfalls bis zur Entnahme des Blutfilters von der Behandlungsvorrichtung. Hiermit einher geht, dass die Fehleranfälligkeit niedrig ist. Zudem hat der Benutzer während der Entleerung Zeit für andere Tätigkeiten. Dies trägt zur Arbeitsentlastung und zur Zeiteinsparung insgesamt bei.

Die vorliegende Erfindung kann ferner in vorteilhafter Weise zum sicheren Entfernen auch von Blut aus dem extrakorporalen Blutkreislauf für eine Behandlungsvorrichtung zur extrakorporalen Blutbehandlung eines Patienten nach Beenden einer Blutbehandlungssitzung eingesetzt werden: Da sich zu gleicher Zeit Luft und Substituatflüssigkeit in Form eines Luft-Substituatflüssigkeit-Blut-Inhalts im Leitungsinneren des extrakorporalen Blutkreislaufs nach Beenden der Blutbehandlungssitzung befinden, kann das im Leitungsinneren des extrakorporalen Blutkreislaufs vorhandene Blut aus dem extrakorporalen Blutkreislauf entfernt werden. Dies kann ohne Gefahr erfolgen, Luft in den Körper des Patienten einzubringen.

Durch Fördern, insbesondere volumendefinierter, Mengen an Luft und Substituatflüssigkeit kann der Inhalt des Leitungsinneren des extrakorporalen Blutkreislaufs über vorgegebene Distanzen im extrakorporalen Blutkreislauf gefördert werden und so ein wesentliches Entfernen von Flüssigkeiten (Blut und ggf. auch Substituatflüssigkeit) aus dem Leitungsinneren des extrakorporalen Blutkreislaufs gewährleistet werden. Dabei bedarf es nur einer geringen Menge an Substituatflüssigkeit. Dies senkt vorteilhaft die Kosten und den Bereitstellungsaufwand für das verwendete Substituat.

Die im Blutschlauchsatz gegebenenfalls zurückbleibende Menge an Substituatflüssigkeit kann beispielsweise dem Volumen des venösen Abschnitts des extrakorporalen Blutkreislaufs entsprechen und beispielsweise nur etwa 30 ml betragen. Damit wird bereits in vorteilhafter Weise zur Verringerung des Abfallgewichts des extrakorporalen Blutkreislaufs beigetragen. Die im Blutschlauchsatz verbleibende - hinsichtlich ihres Beitrags zum Gesamtgewicht des Schlauchsatzes vernachlässigbare - Substituatflüssigkeitsmenge kann in vorteilhafter Weise als Sicherheitspuffer dienen und zu diesem Zweck im Blutschlauchsatz verbleiben. Auf diese Weise kann ein Einbringen von Luft in den Blutkreislauf des Patienten verhindert werden.

Das Einbringen von Substituatflüssigkeit zwischen den Luft-Blut-Inhalt im Leitungsinneren kann in vorteilhafter Weise dazu beitragen, das Eintragen von Luft in das Gefäßsystem des Patienten bei Entleeren des extrakorporalen Blutkreislaufs bei dessen Konnektion mit dem Patienten zu vermeiden, und dennoch können mittels der Substituatflüssigkeit als Spülflüssigkeit Blutrückstände aus dem Leitungsinneren des extrakorporalen Blutkreislaufs gespült und so ein Kontaminationsrisiko weiter ausgeschlossen werden. Der extrakorporale Blutkreislauf kann somit sicher und mit geringem Restgesamtgewicht bzw. Bruttoabfallgewicht entsorgt werden.

Das Einbringen von Substituatflüssigkeit kann ferner dazu beitragen, einen Blutfilter mit verschiedenen Kapillarinnendurchmessern von Blut zu reinigen, was durch lediglich Durchströmen mit Luft aufgrund des geringeren Druckabfalls über den bereits luftgefüllten Kapillaren im Gegensatz zu den noch mit Blut gefüllten Kapillaren technisch schlecht realisierbar wäre.

Daneben kann es auch möglich sein, eine Schaumbildung, z. B. am Ausgang des Blutfilters, zu minimieren, indem dort Substituatflüssigkeit, also insbesondere eine Flüssigkeit, nicht aber allein ein Gas (z. B. Luft) zum Verdrängen von Blut oder Entleeren eingesetzt wird.

Da das erfindungsgemäße Verfahren direkt nach Beenden einer Blutbehandlungssitzung durchgeführt werden kann, ist es einfach und leicht durchzuführen und erfordert keine technisch aufwändigen, zeit- und/oder kostenintensiven Schritte.

Das erfindungsgemäße Verfahren kann in vorteilhafter Weise mit der bei einer Blutbehandlung ohnehin eingesetzten oder vorliegenden Substituatflüssigkeit, wie beispielsweise einer isotonen Kochsalzlösung, z. B. einer 0,9%-igen NaCl-Lösung, durchgeführt werden. Dies trägt wiederum in vorteilhafter Weise zu einer Kosten- und Zeitersparnis bei.

Ferner kann das erfindungsgemäße Verfahren ein Entfernen von Blut aus dem arteriellen Abschnitt des extrakorporalen Blutkreislaufs und insbesondere aus der arteriellen Konnektionsnadel und ein Rückführen desselben in das Gefäßsystem des Patienten ermöglichen. Es kann somit in vorteilhafter Weise der Schritt umgangen werden, das in der arteriellen Konnektionsnadel befindliche Blut mit Hilfe z. B. einer mit Kochsalzlösung aufgezogenen Spritze retrograd herauszudrücken.

Das erfindungsgemäße Verfahren kann somit den Vorteil bieten, das im Leitungsinneren eines extrakorporalen Blutkreislaufs nach dessen Verwendung bei einer Blutbehandlung vorhandene Blut im Wesentlichen vollständig für den Patienten zurückzugewinnen.

Die erfindungsgemäße Vorgehensweise stellt sicher, dass beim Entleeren keine Luft in das Gefäßsystem des Patienten eintritt. Ferner kann es bei diesem Verfahren zu keiner Schaumbildung im Bereich eines im extrakorporalen Blutkreislauf vorhandenen Blutfilters kommen, was ein Entleeren des Bluts aus dem extrakorporalen Blutkreislauf erschweren würde. Im extrakorporalen Blutkreislauf verbleibendes Blut bildet aber wiederum ein Kontaminationsrisiko.

Ein weiterer, in einigen erfindungsgemäßen Ausführungsformen erzielbarer Vorteil besteht darin, dass erstmals ein einfaches und sicheres Verfahren vorgeschlagen wird, mit welchem das Restgewicht nach Beendigung der Blutbehandlung sowohl des Blutfilters als auch des Blutkreislaufs beachtlich verringert werden kann. So wird erfindungsgemäß aufgezeigt, wie sowohl Blutfilter als auch Blutkreislauf geleert werden, wobei dies in manchen Ausführungsformen ganz oder nahezu vollständig automatisch, jedenfalls aber mit geringer Tätigkeit des medizinischen Personals erfolgen kann. Dies wird nicht zuletzt durch die optimierte zeitliche Abfolge des Entleerens von Dialysatkammer einerseits und des Entleerens der übrigen, in manchen erfindungsgemäßen Ausführungsformen ebenfalls entleerten Abschnitte des Blutfilters und des Blutkreislaufs andererseits erzielt.

Im Folgenden wird das erfindungsgemäße Verfahren anhand bevorzugter Ausführungsformen desselben unter Bezugnahme auf die angehängte Zeichnung beschrieben. In der Zeichnung gilt:

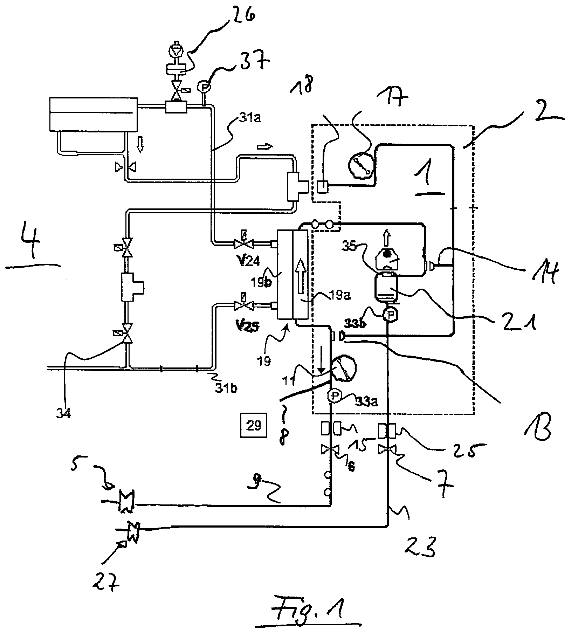

- Fig. 1: zeigt eine schematische Übersicht einer beispielhaften Anordnung als Teil einer Blutkassette zur Durchführung des erfindungsgemäßen Verfahrens.

Fig. 1 zeigt einen extrakorporalen Blutkreislauf 1, welcher mittels Double-Needle-Zugang mit dem Gefäßsystem des nicht dargestellten Patienten verbunden ist. Der Blutkreislauf 1 liegt in Abschnitten hiervon in oder auf einer Blutkassette 2 vor. Er ist mit einer Behandlungsvorrichtung 4 verbunden.

Der Blutkreislauf 1 weist eine arterielle Patientenschlauchklemme 6 und eine arterielle Konnektionsnadel 5 (als Beispiel einer Zugangseinrichtung) eines arteriellen Abschnitts oder einer arteriellen Blutleitung 9 auf. Der Blutkreislauf 1 weist ferner eine venöse Patientenschlauchklemme 7 und eine venöse Konnektionsnadel 27 (als Beispiel einer weiteren oder zweiten Zugangseinrichtung) eines venösen Abschnitts oder einer venösen Blutleitung 23 auf.

Eine Blutpumpe 11 ist im arteriellen Abschnitt 9 vorgesehen, eine Substituatpumpe 17 ist mit einer Substituatleitung verbunden. Die Substituatleitung kann mittels eines automatischen Substituatports 18 mit einer Substituatquelle verbunden werden. Mittels der Substituatpumpe 17 kann Substituat per Prädilution oder per Postdilution und zugehörigen Leitungen 13 bzw. 14 in Leitungsabschnitte des Blutkreislaufs 1 eingebracht werden.

In den Blutkreislauf 1 ist ein Blutfilter 19 eingeschaltet. Dieser weist eine mit dem arteriellen Abschnitt 9 und mit dem venösen Abschnitt 23 verbundene Blutkammer 19a auf. Eine Dialysatkammer 19b ist mit einer zur Dialysatkammer 19b führenden Dialysatzulaufleitung 31a und einer von der Dialysatkammer 19b fortführenden Dialysatablaufleitung 31b verbunden.

Die Dialysatzulaufleitung 31a weist ein Ventil V24 auf, mittels welchem der Fluss innerhalb der Dialysatzulaufleitung 31a unterbunden werden kann. Die Dialysatablaufleitung 31b weist ein Ventil V25 auf, mittels welchem der Fluss innerhalb der Dialysatablaufleitung 31b unterbunden werden kann.

Die Dialysatzulaufleitung 31a ist ferner mittels eines weiteren, maschineninternen Ventils mit einer Druckluftquelle 26 verbunden.

Zur Durchführung des erfindungsgemäßen Verfahrens mit dem Ziel, zunächst die Dialysatkammer 19b des Blutfilters 19 nach Ende der Behandlung zu leeren, bleiben die Dialysatzulaufleitung 31a und die Dialysatablaufleitung 31b jeweils mit dem Blutfilter 19 verbunden. An der Dialysatablaufleitung 31b wird über das Ventil V25 ein Unterdruck angelegt. Alternativ oder ergänzend wird über das maschineninterne Ventil V24 hinweg Luft in die Dialysatkammer 19b eingebracht. Die eingebrachte Luft kann Druckluft aus der Druckluftquelle 26 sein. Die Druckluftquelle 26 kann Bestandteil der Behandlungsvorrichtung 4 sein oder getrennt hiervon vorliegen.

Die eingebrachte Luft kann alternativ hierzu Atmosphärenluft sein, die durch Öffnen der entsprechenden Ventile oder anderer Bauteile in die Dialysatzulaufleitung 31a eindringen kann.

Der Zustrom von Luft oder eines anderen Gases kann mittels eines oder mehrerer Sensoren, insbesondere Drucksensoren, geregelt werden. Der oder die Sensoren können am Dialysatzu- und/oder -ablauf vorgesehen sein. Insbesondere können sie in der Dialysatzulaufleitung 31a und/oder der Dialysatablaufleitung 31b vorgesehen sein, wie dies am Beispiel eines Drucksensors 37 in Fig. 1 gezeigt ist.

Um ein Verdrängen von Dialysat aus der Dialysatkammer 19b über die Membran in die Blutkammer 19a hinein zu verhindern, wie dies in bestimmten erfindungsgemäße Ausführungsformen beabsichtigt ist, kann es von Vorteil sein, die Blutkassette 2 oder den extrakorporalen Blutkreislauf 1 auf der Blutseite gegen die Atmosphäre verschlossen zu halten oder zu verschließen. Dies kann bei Verwenden der in Fig. 1 dargestellten Anordnung beispielsweise durch Schließen der arteriellen und venösen Patientenschlauchklemmen 6 und 7 sowie des Single-Needle-Ventils 35 erfolgen. Alternativ kann ein Flüssigkeitsübertritt auf die Blutseite der Membran durch geeignete Wahl der Betriebsdrücke vermieden werden. Dabei kann der Fluiddruck, welcher auf der Blutseite der Membran herrscht, größer sein oder höher eingestellt werden als auf der Dialysatseite. Der auf der Blutseite herrschende Druck kann beispielsweise mittels eines oder mehrerer im extrakorporalen Blutkreislauf 1 und insbesondere im arteriellen und/oder im venösen Abschnitt vorgesehenen Drucksensors/en 33a, 33b gemessen werden.
Soll ferner auch noch der extrakorporale Blutkreislauf 1 und/oder die Blutkammer 19a von Fluid, d. h. Blut, geleert werden, so kann exemplarisch wie folgt vorgegangen werden:
Der extrakorporale Blutkreislauf 1 wird vom Gefäßsystem des Patienten dekonnektiert, indem die arterielle Konnektionsnadel 5 aus dem Arm des Patienten gezogen wird.

Zum Starten des erfindungsgemäßen Verfahrens in einer ersten Ausführungsform wird zunächst die venöse Patientenschlauchklemme 7 geöffnet. Anschließend wird die Blutpumpe 11 gestartet und so Luft in den arteriellen Abschnitt 9 des extrakorporalen Blutkreislaufs 1 gesaugt. Die Blutpumpe 11 ist exemplarisch als Rollenpumpe ausgebildet und trägt über das dekonnektierte Ende ein - z. B. vorbestimmtes - Volumen an Luft in den extrakorporalen Blutkreislauf 1 ein. Anschließend wird der Luft-Blut-Inhalt entlang des Leitungsinneren des extrakorporalen Blutkreislaufs 1 durch Betreiben der Blutpumpe 11 in Richtung zur venösen Konnektionsnadel 27 gefördert.

Das vorbestimmte Volumen an Luft ist in dieser Ausführungsform so definiert, dass die Luft-Blut-Grenze im Leitungsinneren des extrakorporalen Blutkreislaufs 1 möglichst genau vor der Zugabestelle 13 für Substituatflüssigkeit zum Stehen kommt. Um die Genauigkeit des Stoppens der Luft-Blut-Grenze vor oder an der Zugabestelle 13 für Substituatflüssigkeit zu erhöhen, kann ein arterieller Luft-Blut-Detektor 15 als ein Beispiel einer Erfassungseinrichtung an geeigneter Stelle im arteriellen Abschnitt 9 des extrakorporalen Blutkreislaufs 1 zwischen der Zugabestelle 13 für Substituatflüssigkeit und der arteriellen Konnektionsnadel 5 positioniert sein.

Wenn die Luft-Blut-Grenze die Zugabestelle 13 für Substituatflüssigkeit erreicht, wird die Blutpumpe 11 gestoppt. Die zweite Fördereinrichtung, die hier exemplarisch als Rollenpumpe ausgebildete Substituatpumpe 17, trägt ein - bevorzugt - vorgegebenes Volumen an Substituatflüssigkeit in das Leitungsinnere des extrakorporalen Blutkreislaufs 1 über die Zugabestelle 13 für Substituatflüssigkeit ein. Die Substituatpumpe 17 kann anschließend gestoppt werden.

Alternativ kann die Substituatflüssigkeit auch durch Betreiben der Blutpumpe 11 eingebracht werden. Dazu wird die arterielle Patientenschlauchklemme 6 geschlossen und Substituatflüssigkeit über eine Zuleitung 8 aus einem Vorratsbehälter für die Substituatflüssigkeit in den extrakorporalen Blutkreislauf 1 eingebracht.

Der entstandene Luft-Substituatflüssigkeit-Blut-Inhalt wird durch erneutes Betreiben der Blutpumpe 11 entlang des Leitungsinneren des extrakorporalen Blutkreislaufs 1 gefördert und durch den Blutfilter 19, die venöse Luftabscheidekammer 21 und einen venösen Abschnitt 23 des extrakorporalen Blutkreislaufs 1 gedrückt bzw. gefördert, um so das Blut aus dem extrakorporalen Blutkreislauf 1 in Richtung zur venösen Konnektionsnadel 27 zu entfernen. Dabei kann wiederum - durch die Blutpumpe 11 und/oder die Substituatpumpe 17 - Luft eingebracht werden.

Im venösen Abschnitt 23 des extrakorporalen Blutkreislaufs 1 ist ein venöser Luft-Substituatflüssigkeit-Blut-Detektor 25 als ein weiteres Beispiel einer Erfassungseinrichtung angeordnet, der das Auftreten von Substituatflüssigkeit an einer vorbestimmten Position des Leitungsinneren des extrakorporalen Blutkreislaufs 1 erkennt. Die Blutpumpe 11 fördert den Luft-Substituatflüssigkeit-Blut-Inhalt solange weiter, bis das Blut im venösen Abschnitt 23 des extrakorporalen Blutkreislaufs 1 aus selbigem entfernt und über die venöse Konnektionsnadel 27 in das Gefäßsystem des Patienten rückgeführt worden ist und/oder bis am venösen Luft-Substituatflüssigkeit-Blut-Detektor 25 das Auftreten von Luft im Leitungsinneren erfasst wird. Die Förderleistung aller Pumpen wird beendet. Es kann ein optisches und/oder akustisches Signal ausgegeben werden.

Die Steuerung oder Regelung der Behandlungsvorrichtung 4 kann mittels einer Steuerungs- oder Regelungseinrichtung 29 erfolgen.

Eine zweite Ausführungsform der vorliegenden Erfindung entspricht im Wesentlichen der vorstehend beschriebenen ersten Ausführungsform, außer, dass der Luft-Substituatflüssigkeit-Blut-Inhalt im Leitungsinneren des extrakorporalen Blutkreislaufs 1 nach Zugeben der Substituatflüssigkeit nicht durch Betreiben der Blutpumpe 11, sondern durch Betreiben der Substituatpumpe 17 entlang des Leitungsinneren des extrakorporalen Blutkreislaufs 1 gefördert wird.

Die vorliegende Erfindung ist nicht auf die vorstehend beschriebenen Ausführungsformen beschränkt, diese dienen lediglich der Veranschaulichung. Auch ist die Erfindung nicht auf ein Entleeren des Inhalts oder Teile hiervon bei bestehender Konnektierung mit dem Gefäßsystem beschränkt.

### Bezugszeichenliste

- 1: extrakorporaler Blutkreislauf
- 2: Blutkassette
- 4: Behandlungsvorrichtung
- 5: Zugangseinrichtung, beispielsweise arterielle Konnektionsnadel
- 6: arterielle Patientenschlauchklemme
- 7: venöse Patientenschlauchklemme
- 8: Zuleitung
- 9: arterieller Abschnitt oder arterielle Blutleitung
- 11: Blutpumpe
- 13: Zugabestelle für Substituatflüssigkeit (Prädilution)
- 14: Zugabestelle für Substituatflüssigkeit (Postdilution)
- 15: arterieller Luft-Blut-Detektor
- 17: zweite Fördereinrichtung, beispielsweise eine Substituatpumpe
- 18: automatischer Substituatport
- 19: Blutfilter
- 19a: Blutkammer
- 19b: Dialysatkammer
- 21: venöse Luftabscheidekammer
- 23: venöser Abschnitt oder venöse Blutleitung
- 25: venöser Luft-Substituatflüssigkeit-Blut-Detektor
- 26: Druckluftquelle
- 27: Zugangseinrichtung, beispielsweise venöse Konnektionsnadel
- 29: Steuerungs- oder Regelungseinrichtung
- 31a: Dialysatzulaufleitung
- 31b: Dialysatablaufleitung
- 33a, b: Drucksensoren
- 35: Single-Needle-Ventil
- 37: Drucksensor
- V24: Ventil
- V25: Ventil

## Patentansprüche

1. Steuerungs- und/oder Regelungseinrichtung (29), konfiguriert, um die Durchführung eines Verfahren zum Entfernen von Fluid aus einem zur Blutbehandlung eines Patienten verwendeten Blutfilter (19) oder Blutkreislauf (1) nach Beendigung der Blutbehandlungssitzung, zu steuern oder zu regeln,
wobei der Blutfilter (19) eine Blutkammer (19a) und eine Dialysatkammer (19b) aufweist, zwischen welchen eine Membran angeordnet ist, wobei die Blutkammer (19a) zur Blutbehandlung mit einer zur Blutkammer (19a) führenden arteriellen Blutleitung (9) und einer von der Blutkammer (19a) weg führenden venösen Blutleitung (23) sowie einer zur Dialysatkammer (19b) führenden Dialysatzulaufleitung (31a) und einer von der Dialysatkammer (19b) weg führenden Dialysatablaufleitung (31b) verbunden ist, wobei das Verfahren die Schritte umfasst:
- Verdrängen des Fluides aus der Dialysatkammer (19b) durch aktives oder passives Einbringen von Luft oder einer Flüssigkeit in die Dialysatzulaufleitung (31a), und
- wobei ferner Blut aus der Blutkammer (19a) des Blutfilters (19) und/oder aus einem mit dem Blutfilter (19) verbundenen extrakorporalen Blutkreislauf (1), welcher ein Leitungsinneres aufweist, entfernt wird, mittels
- wenigstens einer Blutpumpe (11) der Behandlungsvorrichtung (4) zum Fördern von Blut innerhalb des Leitungsinneren des extrakorporalen Blutkreislaufs (1);
- wenigstens einer zweiten Fördereinrichtung (17) zum Einbringen wenigstens einer Substituatflüssigkeit in das Leitungsinnere des extrakorporalen Blutkreislaufs (1) und/oder Fördern eines Leitungsinhalts innerhalb des Leitungsinneren des extrakorporalen Blutkreislaufs (1);
mit den Schritten:
- Eintragen von Luft in das Leitungsinnere des extrakorporalen Blutkreislaufs (1) durch Betreiben der Blutpumpe (11); und
- Einbringen von Substituatflüssigkeit in das Leitungsinnere des extrakorporalen Blutkreislaufs (1) durch Betreiben der zweiten Fördereinrichtung (17),
wobei das Entleeren der Dialysatkammer (19b) vor odergleichzeitig mit dem Entleeren der Blutkammer (19a) oder des Blutkreislaufs (1) erfolgt.

2. Steuerungs- und/oder Regelungseinrichtung (29) nach Anspruch 1, wobei das Verfahren folgenden Schritt aufweist:
- Regeln oder Steuern des Einbringens von Luft unter Berücksichtigung von mittels wenigstens eines in oder an der Dialysatzulaufleitung (31a) oder in oder an der Dialysatablaufleitung (31b) vorgesehenen Drucksensors (37) gemessenen Druckwerten.

3. Steuerungs- und/oder Regelungseinrichtung (29) nach Anspruch 1 oder 2, wobei das Verfahren folgenden Schritt aufweist:
- Verschließen der arteriellen Blutleitung (9) und/oder der venösen Blutleitung (23) gegenüber der Atmosphäre oder Gewährleisten eines solchen Verschlusses.

4. Steuerungs- und/oder Regelungseinrichtung (29) nach einem der vorangegangenen Ansprüche, wobei das Verfahren folgenden Schritt aufweist:
- Festlegen eines in der Blutkammer (19a) herrschenden Drucks auf einen oberhalb eines in der Dialysatkammer (19b) herrschenden Dialysatdrucks gelegenen Druckwert.

5. Steuerungs- und/oder Regelungseinrichtung (29) nach einem der vorhergehenden Ansprüche, wobei die Behandlungsvorrichtung (4) wenigstens eine in einem Abschnitt des extrakorporalen Blutkreislaufs (1) angeordnete Erfassungseinrichtung (15, 25) aufweist, mit dem Schritt:
- Erfassen einer qualitativen Änderung des Inhalts des Leitungsinneren des extrakorporalen Blutkreislaufs (1) mittels der Erfassungseinrichtung (15, 25).

6. Steuerungs- und/oder Regelungseinrichtung (29) nach einem der vorangegangenen Ansprüche, wobei der extrakorporale Blutkreislauf (1) wenigstens eine mit einem Abschnitt des Gefäßsystems des Patienten konnektierbare Zugangseinrichtung (5) aufweist und das Verfahren ferner den Schritt umfasst:
- Dekonnektieren des extrakorporalen Blutkreislaufs (1) vom Gefäßsystem des Patienten.

7. Steuerungs- und/oder Regelungseinrichtung (29) nach einem der vorangegangenen Ansprüche, wobei die Blutpumpe (11) ein definiertes Volumen an Luft fördert.

8. Steuerungs- und/oder Regelungseinrichtung (29) nach einem der vorangegangenen Ansprüche, wobei ein "Luft-Blut-Inhalt" im Leitungsinneren des extrakorporalen Blutkreislaufs (1) gefördert wird, bis eine "Luft-Blut-Grenze" eine Zugabestelle (13, 14) des extrakorporalen Blutkreislaufs (1) für Substituatflüssigkeit in das Leitungsinnere erreicht, und wobei bei Erreichen eine Substituatflüssigkeit über die Zugabestelle (13, 14) in das Leitungsinnere des extrakorporalen Blutkreislaufs (1) eingebracht wird.

9. Steuerungs- und/oder Regelungseinrichtung (29) nach einem der vorangegangenen Ansprüche, wobei durch Betreiben der zweiten Fördereinrichtung (17) eine vorbestimmte Substituatflüssigkeitsmenge in das Leitungsinnere des extrakorporalen Blutkreislaufs (1) eingebracht wird.

10. Steuerungs- und/oder Regelungseinrichtung (29) nach einem der vorangegangenen Ansprüche, wobei die unter Erzeugen eines "Luft-Substituatflüssigkeit-Blut-Inhalts" in Richtung weg von einem dekonnektierten Ende des extrakorporalen Blutkreislaufs (1) unter Betreiben der zweiten Fördereinrichtung (17) zugeführte Substituatflüssigkeit gefördert wird, bis die Erfassungseinrichtung (25) Substituatflüssigkeit im Leitungsinneren des extrakorporalen Blutkreislaufs (1) erfasst.

11. Steuerungs- und/oder Regelungseinrichtung (29) nach einem der vorangegangenen Ansprüche, wobei der "Luft-Substituatflüssigkeit-Blut-Inhalt" durch Betreiben der Blutpumpe (11) und/oder durch Betreiben der zweiten Fördereinrichtung (17) entlang des Leitungsinneren des extrakorporalen Blutkreislaufs (1) gefördert wird.

12. Steuerungs- und/oder Regelungseinrichtung (29) nach einem der vorangegangenen Ansprüche, wobei mittels der zweiten Fördereinrichtung (17) zu wenigstens einem ersten Zeitpunkt Substituatflüssigkeit und zu wenigstens einem sich zeitlich vom ersten Zeitpunkt unterscheidenden zweiten Zeitpunkt Luft in das Leitungsinnere des extrakorporalen Blutkreislaufs (1) eingebracht wird.

13. Steuerungs- und/oder Regelungseinrichtung (29) nach einem der vorgegangenen Ansprüche, wobei die Erfassungseinrichtung (25) mit vorgegebener Distanz zu einer zweiten Zugangseinrichtung (27) angeordnet ist und das Verfahren ferner den Schritt umfasst:
- Fördern des Inhalts des Leitungsinneren des extrakorporalen Blutkreislaufs (1) über die vorgegebene Distanz bis zur Zugangseinrichtung (27), nachdem Luft an der Erfassungseinrichtung (25) erkannt wurde.

14. Medizinische Behandlungsvorrichtung (4) mit
- wenigstens einem extrakorporalen Blutkreislauf (1) mit einem Leitungsinneren;
- wenigstens einer an oder in dem extrakorporalen Blutkreislauf (1) angeordneten Blutpumpe (11) zum Fördern von Blut innerhalb des Leitungsinneren des extrakorporalen Blutkreislaufs (1);
- wenigstens einer Einrichtung zum Einbringen von Luft in eine Dialysatzulaufleitung (31a) zum Zuführen von Dialysat zur Dialysatkammer (19b);
**gekennzeichnet durch**
- wenigstens eine Steuerungs- und/oder Regelungseinrichtung (29) nach einem der Ansprüche 1 bis 13.

15. Vorrichtung nach Anspruch 14 mit
- wenigstens einer zweiten Fördereinrichtung (17) zum Einbringen wenigstens einer Substituatflüssigkeit in das Leitungsinnere des extrakorporalen Blutkreislaufs (1) und/oder Fördern eines Leitungsinhalts innerhalb des Leitungsinneren des extrakorporalen Blutkreislaufs (1), und/oder
- wenigstens einer in oder an einem Abschnitt des extrakorporalen Blutkreislaufs (1) angeordneten Erfassungseinrichtung (15, 25) zum Erfassen wenigstens einer Änderung des Inhalts oder einer Eigenschaft des Inhalts des Leitungsinneren.

## Claims

1. A control and/or regulating device (29), configured for controlling or regulating the execution of a method for removing fluid from a blood filter (19) or blood circuit (1) used for a blood treatment of a patient after the end of the blood treatment session,
wherein the blood filter (19) comprises a blood chamber (19a) and a dialysate chamber (19b) between which a membrane is arranged, wherein for purposes of the blood treatment the blood chamber (19a) is connected with an arterial blood line (9) which leads to the blood chamber (19a) and with a venous blood line (23) which leads away from the blood chamber (19a) as well as with a dialysate inlet line (31a) which leads to the dialysate chamber (19b) and with a dialysate outlet line (31b) which leads away from the dialysate chamber (19b), wherein the method encompasses the steps:
- displacing the fluid from the dialysate chamber (19b) by actively or passively introducing air or a liquid into the dialysate inlet line (31a), and
- wherein blood is removed from the blood chamber (19a) of the blood filter (19) and/or from an extracorporeal blood circuit (1) which is connected with the blood filter (19) and which comprises a line interior, by means of
- at least one blood pump (11) of the treatment apparatus (4) for conveying blood within the line interior of the extracorporeal blood circuit (1);
- at least one second conveying device (17) for introducing at least one substituate liquid into the line interior of the extracorporeal blood circuit (1) and/or conveying a line content within the line interior of the extracorporeal blood circuit (1);
with the steps:
- introducing air into the line interior of the extracorporeal blood circuit (1) by operating the blood pump (11); and
- introducing substituate liquid into the line interior of the extracorporeal blood circuit (1) by operating the second conveying device (17),
wherein emptying the dialysate chamber (19b) is carried out before or at the same time of emptying the blood chamber (19a) or the blood circuit (1).

2. The control and/or regulating device (29) according to claim 1, wherein the method comprises the following step:
- regulating or controlling the introduction of air while considering pressure measures measured by means of at least one pressure sensor (37) provided in or on the dialysate inlet line (31a) or in or on the dialysate outlet line (31b).

3. The control and/or regulating device (29) according to claim 1 or 2, wherein the method comprises the following step:
- sealing the arterial blood line (9) and/or the venous blood line (23) against the atmosphere or ensuring such sealing.

4. The control and/or regulating device (29) according to anyone of the preceding claims, wherein the method comprises the following step:
- setting a pressure present in the blood chamber (19a) to a pressure value which is above a dialysate pressure present in the dialysate chamber (19b).

5. The control and/or regulating device (29) according to anyone of the preceding claims, wherein the treatment apparatus (4) comprises at least one detection device (15, 25) which is arranged in a section of the extracorporeal blood circuit (1), with the step:
- recording a qualitative change of the line interior content of the extracorporeal blood circuit (1) by means of the detection device (15, 25).

6. The control and/or regulating device (29) according to any one of the preceding claims, wherein the extracorporeal blood circuit (1) comprises at least one access device (5) which is connectable with a section of the patient's vascular system, and wherein the method further encompasses the step:
- disconnecting the extracorporeal blood circuit (1) from the patient's vascular system.

7. The control and/or regulating device (29) according to anyone of the preceding claims, wherein the blood pump (11) conveys a defined volume of air.

8. The control and/or regulating device (29) according to anyone of the preceding claims, wherein an *air-blood content* in the line interior of the extracorporeal blood circuit (1) is conveyed until an *air-blood boundary* reaches an addition point (13, 14) of the extracorporeal blood circuit (1) for substituate liquid in the line interior, and wherein substituate liquid is introduced into the line interior of the extracorporeal blood circuit (1) via the addition point (13, 14) when the addition point is reached.

9. The control and/or regulating device (29) according to any one of the preceding claims, wherein a predetermined amount of substituate liquid is introduced into the line interior of the extracorporeal blood circuit (1) by operating the second conveying device (17).

10. The control and/or regulating device (29) according to anyone of the preceding claims, wherein the substituate liquid, which was supplied by generating an *air-substituate liquid-blood content*, is conveyed in the direction which leads away from a disconnected end of the extracorporeal blood circuit (1) by operating the second conveying device (17) until the detection device (25) detects substituate liquid in the line interior of the extracorporeal blood circuit (1).

11. The control and/or regulating device (29) according to anyone of the preceding claims, wherein the *air-substituate liquid-blood content* is conveyed along the line interior of the extracorporeal blood circuit (1) by operating the blood pump (11) and/or by operating the second conveying device (17).

12. The control and/or regulating device (29) according to any one of the preceding claims, wherein, by means of the second conveying device (17), substituate liquid is introduced into the line interior of the extracorporeal blood circuit (1) at at least a first point of time and air is introduced into the line interior of the extracorporeal blood circuit (1) at at least a second point of time which differs from the first point of time.

13. The control and/or regulating device (29) according to anyone of the preceding claims, wherein the detection device (25) is arranged with a predetermined distance to a second access device (27), and wherein the method further comprises the step:
- conveying the content of the line interior of the extracorporeal blood circuit (1) across the predetermined distance to the access device (27) after air was detected at the detection device (25).

14. A medical treatment apparatus (4) having
- at least one extracorporeal blood circuit (1) with a line interior;
- at least one blood pump (11) which is arranged at or in the extracorporeal blood circuit (1) for conveying blood within the line interior of the extracorporeal blood circuit (1);
- at least one device for introducing air into a dialysate inlet line (31a) for supplying dialysate to the dialysate chamber (19b);
**characterized by**
- at least one control and/or regulating device (29) according to any one of the claims 1 to 13.

15. The apparatus according to claim 14 with
- at least one second conveying device (17) for introducing at least one substituate liquid into the line interior of the extracorporeal blood circuit (1) and/or conveying a line content within the line interior of the extracorporeal blood circuit (1)
- and/or at least one detection device (15, 25) arranged in or at a section of the extracorporeal blood circuit (1) for detecting at least one change of the content or of a property of the content of the line interior.

## Revendications

1. Un dispositif de commande ou de régulation (29) configuré pour commander ou réguler l'exécution d'un procédé d'élimination du fluide d'un filtre sanguin (19) ou d'un circuit sanguin (1) utilisé pour le traitement du sang d'un patient après la fin d'une séance de traitement du sang,
le filtre sanguin (19) comprenant une chambre à sang (19a) et une chambre de dialysat (19b) entre lesquelles est disposée une membrane, la chambre à sang (19a) étant reliée pour le traitement à une ligne de sang artérielle (9) conduisant à la chambre à sang (19a) et à une ligne de sang veineuse (23) sortant de la chambre à sang (19a) ainsi qu'à une ligne d'entrée du dialysat (31a) conduisant à la chambre de dialysat (19b) et à une ligne de sortie du dialysat (31b) sortant de la chambre de dialysat (19b), le procédé comprenant les étapes consistant à :
- déplacer le fluide de la chambre de dialysat (19b) par introduction active ou passive d'air ou de liquide dans la ligne d'entrée de dialysat (31a), et
- où le sang est retiré de la chambre à sang (19a) du filtre sanguin (19) et/ou du circuit sanguin extracorporel (1) relié au filtre sanguin (19) comprenant une ligne ayant une partie intérieure, en utilisant
- au moins une pompe sanguine (11) de l'appareil de traitement (4) pour transporter le sang à l'intérieur de la partie intérieure de la ligne du circuit sanguin extracorporel (1);
- au moins un second dispositif de transport (17) prévu pour introduire au moins un liquide de substitution dans la partie intérieure de la ligne du circuit sanguin extracorporel (1) et/ou transporter un contenu de ligne dans la partie intérieure de la ligne du circuit sanguin extracorporel (1);
avec les étapes suivantes :
- introduire de l'air dans la partie intérieure de la ligne du circuit sanguin extracorporel (1) en actionnant la pompe à sang (11); et
- introduire un liquide de substitution dans la partie intérieure de la ligne du circuit sanguin extracorporel (1) en actionnant le second dispositif de transport (17),
où la vidange de la chambre de dialysat (19b) est effectuée avant ou au même moment que la vidange de la chambre à sang (19a) ou du circuit sanguin (1).

2. Le dispositif de commande ou de régulation (29) selon la première revendication, où le procédé comprend l'étape suivante :
- réguler ou commander l'introduction d'air tout en considérant des valeurs de pression mesurées au moyen d'au moins un capteur de pression (37) prévu dans ou sur la ligne d'entrée du dialysat (31a) ou dans ou sur la ligne de sortie de dialysat (31b).

3. Le dispositif de commande ou de régulation (29) selon la première ou la seconde revendication, où le procédé comprend l'étape suivante :
- étanchéifier la ligne sanguine artérielle (9) et/ou la ligne sanguine veineuse (23) contre l'atmosphère ou assurer une telle étanchéité.

4. Le dispositif de commande ou de régulation (29) selon l'une quelconque des revendications précédentes, où le procédé comprend l'étape suivante :
- établir une pression s'appliquant dans la chambre à sang (19a) à une valeur de pression supérieure à une pression du dialysat s'appliquant dans la chambre de dialysat (19b).

5. Le dispositif de commande ou de régulation (29) selon l'une quelconque des revendications précédentes, où l'appareil de traitement (4) comprend au moins un dispositif de détection (15, 25) agencé dans une section du circuit sanguin extracorporel (1), comprenant l'étape consistant à :
- saisir un changement qualitatif du contenu de la partie intérieure de la ligne du circuit sanguin extracorporel (1) au moyen du dispositif de détection (15, 25).

6. Le dispositif de commande ou de régulation (29) selon l'une quelconque des revendications précédentes, où le circuit sanguin extracorporel (1) comprend au moins un dispositif d'accès (5) pouvant être connecté à une section du système vasculaire du patient, le procédé comprenant en outre l'étape consistant à :
- déconnecter le circuit sanguin extracorporel (1) du système vasculaire du patient.

7. Le dispositif de commande ou de régulation (29) selon l'une quelconque des revendications précédentes, où la pompe à sang (11) transporte un volume défini d'air.

8. Le dispositif de commande ou de régulation (29) selon l'une quelconque des revendications précédentes, où un « contenu air/sang » est transporté dans la partie intérieure de ligne du circuit sanguin extracorporel (1) jusqu'à ce qu'une « limite air/sang » atteigne un point d'addition (13, 14) de liquide de substitution du circuit sanguin extracorporel (1), et où un liquide de substitution est introduit dans la partie intérieure de la ligne du circuit sanguin extracorporel (1) via le point d'addition (13, 14) lorsque le point d'addition est atteint.

9. Le dispositif de commande ou de régulation (29) selon l'une quelconque des revendications précédentes, où une quantité prédéterminée de liquide de substitution est introduite dans la partie intérieure de la ligne du circuit de sang extracorporel (1) en actionnant le second dispositif de transport (17).

10. Le dispositif de commande ou de régulation (29) selon l'une quelconque des revendications précédentes, où le liquide de substitution introduit par la génération du « contenu air/liquide de substitution/sang » en actionnant le second dispositif de transport (17) est transporté dans une direction s'éloignant d'une extrémité déconnectée du circuit sanguin extracorporel (1) jusqu'à ce que le dispositif de détection (25) détecte du liquide de substitution dans la partie intérieure de la ligne du circuit sanguin extracorporel (1).

11. Le dispositif de commande ou de régulation (29) selon l'une quelconque des revendications précédentes, où le « contenu air/liquide de substitution/sang » est transporté le long de la partie intérieure de la ligne du circuit sanguin extracorporel (1) en actionnant la pompe à sang (11) et/ou en actionnant le second dispositif de transport (17).

12. Le dispositif de commande ou de régulation (29) selon l'une quelconque des revendications précédentes, où, au moyen du second dispositif de transport (17), du liquide de substitution est introduit dans la partie intérieure de la ligne du circuit sanguin extracorporel (1) à au moins un premier instant et où, au moins à un second instant différent du premier instant, de l'air est introduit dans la partie intérieure de la ligne du circuit sanguin extracorporel (1).

13. Le dispositif de commande ou de régulation (29) selon l'une quelconque des revendications précédentes, où le dispositif de détection (25) est agencé à une distance prédéterminée d'un second dispositif d'accès (27), et où le procédé comprend en outre l'étape consistant à :
- transporter le contenu de la partie intérieure de la ligne du circuit sanguin extracorporel (1) sur la distance prédéterminée jusqu'au second dispositif d'accès (27) après que de l'air ait été détecté par le dispositif de détection (25).

14. Appareil de traitement médical (4) comprenant :
- au moins un circuit sanguin extracorporel (1) avec une ligne ayant une partie intérieure;
- au moins une pompe à sang (11) agencée dans ou sur le circuit sanguin extracorporel (1) pour transporter du sang à l'intérieur de la partie intérieure de la ligne du circuit sanguin extracorporel (1);
- au moins un dispositif pour introduire de l'air dans une ligne d'entrée de dialysat (31a) prévue pour introduire du dialysat dans la chambre de dialysat (19b) ;
**caractérisé par**
- au moins un dispositif de commande ou de régulation (29) selon l'une quelconque des revendications 1 à 13.

15. L'appareil selon la revendication 14, comprenant
- au moins un second dispositif de transport (17) pour introduire au moins un liquide de remplacement dans la partie intérieure de la ligne du circuit sanguin extracorporel (1) et/ou pour transporter un contenu de la ligne à l'intérieur de la partie intérieure de la ligne du circuit sanguin extracorporel (1)
- et/ou au moins un dispositif de détection (15, 25), agencé dans ou sur une section du circuit sanguin extracorporel (1), prévu pour détecter au moins un changement du contenu ou d'une propriété du contenu de la partie intérieure de la ligne.
